# EUROPEAN PATENT SPECIFICATION

(11) **EP 1 558 220 B1**
(45) Date of publication and mention of the grant of the patent: **10.02.2010**
(21) Application number: 03817619.4
(22) Date of filing: 24.07.2003
(51) Int. Cl.: A61K 9/20, A61K 9/22, A61K 9/24, A61K 9/48, A61K 49/00, A61P 3/10

(54) **ORAL COMPOSITIONS FOR TREATMENT OF DIABETES**
ORALE ZUSAMMENSETZUNGEN ZUR BEHANDLUNG VON DIABETES
COMPOSITIONS ORALES POUR LE TRAITEMENT DU DIABETE

(43) Date of publication of application: 03.08.2005
(73) Proprietor: Wockhardt Limited, Mumbai 400 051, Maharashtra (IN); Iyer, Eswaran Krishnan, Mumbai 400 051, Maharashtra (IN); Saoji, Dilip Gopalkrishna, Aurangabad 431 210, Maharashtra (IN); Jha, Rasendrakumar, 400 051 Mumbai, Maharashtra State (IN)
(72) Inventor: IYER, Eswaran Krishnan, Wockhardt Limited, Mumbai 400 051, Maharashtra (IN); SAOJI, Dilip Gopalkrishna, Wockhardt Limited, Aurangabad 431 210, Maharashtra (IN); JHA, Rasendrakumar Jahantilal, Wockhardt Limited, Mumbai 400 051, Maharashtra (IN)
(74) Representative: Kuhnen & Wacker
(86) International application number: PCT/IB2003/002949
(87) International publication number: WO 2005/009412

(56) References cited:
- WO-A-00/27401
- WO-A-01/32158
- WO-A-02/05795
- WO-A-02/26729
- WO-A-02/064210
- WO-A-02/094285
- US-A- 6 011 049
- US-A1- 2002 037 928
- US-A1- 2002 147 157
- US-A1- 2003 077 297
- US-A1- 2003 077 335
- YALE J-F ET AL: "TROGLITAZONE EFFICAY AND SAFETY IN COMBINATION WITH SULFONYLUREA AND METFORMIN AS TRIPLE THERAPY" DIABETES, NEW YORK, NY, US, vol. 48, no. SUPPL 1, 1999, page A118, XP000878792 ISSN: 0012-1797

## Description

### FIELD OF THE INVENTION

This invention describes a pharmaceutical drug delivery system according to the claims for formulating combination of drugs preferably with water solubility and a limited window of absorption, in combination with another immediate /sustained release drug belonging to a different class in combination with yet another immediate release drug belonging to yet another different class, to provide a dosage form that inherently has a prolonged gastric residence. These novel formulations involving triple combination drugs could be used for the treatment of variety of diseases such as non-insulin dependent diabetes mellitus (NIDDM) for improving glycemic control for diabetic patients during their treatment of tenure with anti-diabetic drugs, diseases of the cardiovascular system wherein a triple combination therapy is beneficial either therapeutically or prophylactically and wherein at least one of the agents in the triple combination is a candidate for the formulation of a sustained release product. It also provides for a delivery system comprising of a minimum of three therapeutic agents used for any beneficial treatment of ailments in humans

### BACKGROUND OF THE INVENTION

Diabetes mellitus of type II is a progressive metabolic disorder with diverse pathologic manifestations and is often associated with lipid metabolism and glycometabolic disorders. The long-term effects of diabetes result from its vascular complications; the microvascular complications of retinopathy, neuropathy and nephropathy and the macrovascular complications of cardiovascular, cerebrovascular and peripheral vascular diseases. Initially, diet and exercise is the mainstay of treatment of type II diabetes. However, these are followed by administration of oral hypoglycemic agents. Current drugs used for managing type II diabetes and its precursor syndromes such as insulin resistance, include classes of compounds, such as, among others, biguanides, glitazones and sulfonylureas.

Biguanides represented principally by metformin, phenformin and buformin, help in the control of blood glucose by inhibiting hepatic glucose production, reducing intestinal absorption of glucose and enhancing peripheral glucose uptake. Biguanides, especially metformin, lowers both basal and post-prandial plasma glucose and thus improves tolerance of glucose in patients. Metformin exerts normoglycemic action with reduced risk of lactic acidosis and is also known to lower blood triglyceride levels. It is therefore a preferred mode of therapy among biguanides.

Glitazones, represented principally by the class of glitazones including, for example, rosiglitazone, troglitazone and pioglitazone, among others, act by increasing the sensitivity of insulin receptors in the body and decreasing peripheral insulin resistance. Glitazones, preferably pioglitazone, stimulate adipogenesis and reduce plasma triglyceride and free fatty acid concentrations. These enhance insulin action at the cellular level but do not stimulate insulin release, nor do they mimic its action.

Sulfonylureas, represented principally by glipizide, glimiperide, glyburide, glibornuride, glisoxepide, gliclazide acetohexamide, chlorpropamide, tolazamide, and tolbutamide, among others, help in controlling or managing NIDDM by stimulating the release of endogenous insulin from the beta cells of the pancreas.

Biguanides, glitazones and sulfonylureas are commercially available in the form of tablets of the individual drugs, either immediate release (IR) formulations or in some cases controlled release (CR) formulations, to be administered orally to patients in need thereof, in protocols calling for the single administration of the individual ingredient. Metformin monotherapy is used as a first line treatment in diabetic patients but may be supplemented with other drugs when the secondary failure of the therapy sets in. The addition of a glitazone and sulfonylurea to the concurrent treatment provides a balance of stimulated release of insulin while ameliorating insulin resistance and thus provides an optimal level of glycemic control unattainable by either medication alone. But, multiple medications such as these for the prophylaxis or treatment of diseases usually result in patient inconvenience and consequently, patient non-compliance to the prescribed dosage regimen. The ease of using combination therapy for multiple medications as opposed to separate administrations of the individual medications has long been recognized in the practice of medicine. Such a therapy provides therapeutic advantage for the benefit of the patient and the clinician. Further, such therapy provides both increased convenience and improved patient compliance resulting form the avoidance of missed doses through patient forgetfulness.

A brief logical profile for such a triple combination based on the pharmacological mechanism of action of the individual classes of drugs is given below:
Insulin resistance and reduced insulin secretion are the two fundamental abnormalities in type 2 diabetic patients. Therefore, reducing insulin resistance or increasing insulin sensitivity and augmenting insulin secretion from beta cells of pancreas are the two major treatment approaches. The tissues most commonly resistant to actions of insulin are liver, skeletal muscles, and adipose tissues. Therefore, treatment strategies directed towards improving the insulin sensitivity of these major tissue helps in overall enhancement of insulin sensitivity. It is known that Pioglitazone plays a major role in improving sensitivity of peripheral tissues like skeletal muscles and adipose tissues whereas Metformin has its primary action on liver. Therefore, the combination therapy with Pioglitazone and Metformin results in synergistic actions to improve insulin sensitivity.

Pioglitazone, a member of the thiazolidinedione class of antidiabetic agents, targets insulin resistance by binding to the transcription factor peroxisome proliferator activated receptor-γ (PPAR-γ), promoting synthesis of glucose transporters. It enhances insulin sensitivity, thereby reducing hyperglycemia, glycosylated haemoglobin (HbA1c), hyperinsulinemia and hypertriglyceridemia.

In contrast, Metformin hydrochloride promotes glucose lowering by reducing hepatic glucose production and gluconeogenesis and by enhancing peripheral glucose uptake. Because Metformin and Pioglitazone act through different mechanisms, their combined use is indicated in patients whose disease is poorly controlled with monotherapy.

Pioglitazone increases body weight. However, since Metformin XL reduces body weight, the therapy with consisting of both these drugs is likely to prevent excessive weight gain in an individual

### Metformin XL (Extended Release)

Metformin XL is a modified release gastro-retentive formulation. By virtue of its gastro-retentive property it releases Metformin gradually in small amounts, which is well absorbed in the upper part of the small intestine and duodenum. Metformin incorporated into the gastro-retentive formulation is released slowly over a prolonged period of 24 hours; hence given once a day.

Metformin XL has distinct advantages over plain Metformin which are as follows:
1. It reduces the number of daily doses and increases patient compliance. As treatment of diabetes is life-long, this aspect is very important from a patient's point of view.
2. Metformin XL, being a modified release preparation can also avoid "dose-loading". This commonly occurs with conventional oral formulations when large doses are given which may cause sudden release and absorption of a large amount of drug.
3. Metformin XL is released in smaller doses in upper part of the small intestine, and hence ensures increased bioavailability and decreased side effects. In contrast, conventional Metformin has lesser bioavailability since its absorption decreases as it passes through the lower part of small intestine.
4. Conventional Metformin has an oral bioavailability of 40 to 60 % and gastrointestinal absorption is apparently complete within 6 hours of ingestion. Plasma t 1/2 is 2 to 6 hours. Hence it has to be given 2 to 3 times a day, whereas Metformin XL being a controlled release "gastro-retentive" formulation, is released in small quantities in upper part of small intestine where the drug is better absorbed and has a prolonged duration of action (24 hours).
5. Metformin XL - the absorption is more dependable and complete as the drug is released gradually mainly in the upper part of small intestine, whereas in Metformin plain the absorption is erratic as Metformin is also absorbed in the latter part of small intestine where absorption is erratic and "non-dependable".
6. Since Metformin XL is released slowly, side effects like flatulence, abdominal discomfort, diarrhoea and lactic acidosis are less unlike plain Metformin.
7. An inverse relationship was observed between the dose ingested and relative absorption with therapeutic doses ranging from 0.5 to 1.5 gms suggesting the involvement of an active, saturable absorption process. Thus an extended release formulation of Metformin can not only optimizes the daily requirement of Metformin, but can also reduce the need of a higher dose.

### Glimepiride / Gliclazide / Glipizide :

It is a new third generation sulfonylurea (SU), which binds to a different protein of the SU receptor than glibenclamide. The advantage of glimepiride is that its hydroxy-metabolite has very little hypoglycemic activity therefore it causes fewer hypoglycemic episodes compared to glibenclamide while achieving similar glycemic control. It is relatively safe in elderly patients with mild renal failure. The other advantage with glimepiride is its long life (9 hour) which allows this drug to be used as once daily treatment (1-6mg/kg). A possible third advantage with this drug is that it has no effect on human vascular ATP-dependent K+ channels. Theoretically it will not have any deleterious effect on vascular responses during ischemia.

Glimepiride, being an insulin secretagouge, makes more insulin available for better utilization by the two insulin sensitizers, Pioglitazone and Metformin and hence enhances the actions of the insulin sensitizers.

### Advantages with Pioglitazone, Metformin and glimepiride combination:

1. The triple combination targets the two major pathological processes, insulin resistance and defective insulin secretion.
2. Increased insulin sensitivity due to synergistic actions of Pioglitazone and Metformin
3. Therapeutic actions of Insulin sensitizers, Pioglitazone and Metformin are enhanced due simultaneous administration of glimepiride
4. Better glycemic control
5. Reduced incidence of side effects due reduced dosage requirements of individual drugs.
6. Once a day administration
7. Improved compliance

Metformin is an antihyperglycemic agent of the biguanide class used in the treatment of non-insulin dependent diabetes mellitus (NIDDM). It is usually marketed in the form of its hydrochloride salt as Glucophage. ^{R}(Bristol Meyers Squibb).

Metformin hydrochloride has intrinsically poor permeability in the lower portion of the gastrointestinal tract leading to absorption almost exclusively in the upper part of the gastrointestinal tract. Its oral bioavailability is in the range of 40 to 60% decreasing with increasing dosage which suggests some kind of saturable absorption process, or permeability / transit time limited absorption. It also has a very high water solubility (>300 mg/ml at 25.degree. C.). This can lead to difficulty in providing a slow release rate from a formulation and problems in controlling the initial burst of drug from such a formulation. These two difficulties are further compounded by the high unit dose, 500 mg per tablet, usually required for metformin hydrochloride.(1997-PDR).

Drugs that have absorption limited to the upper gastrointestinal tract coupled with poor absorption in the distal small intestine, large intestine and colon are usually regarded as inappropriate candidates for formulation into oral controlled delivery systems. This limitation on absorption (for example, in the upper gastrointestinal tract) is referred to as the "absorption window".

The gastrointestinal tract functions to propel ingested material from the stomach (where digestion takes place) into the small intestine (where absorption principally occurs) and on to the large intestine (where water is absorbed / secreted as part of body fluid regulation processes). Residence time for non-digestible materials in the stomach depends on whether one is dealing with a fed or a fasted subject. Typical gastric emptying times for particulate material (greater than a few millimeters in diameter) varies from a few tens of minutes in the fasted state to a few hours in the fed state. Transit times through the small intestine are consistently of the order of 3 to 4 hours.

Oral controlled release delivery systems function by releasing their payload of drug over an extended period of time following administration. Thus, controlled release dosage forms may only spend a relatively short period in the regions of the gastrointestinal tract where good absorption of certain drugs can occur. The dosage form will pass on to regions of the intestine where absorption of certain drugs is poor or non-existent, still releasing its contained drug albeit with a significant percentage of its payload still to be delivered. Drug when released from the dosage form in the circumstances described will not be absorbed. Thus, administration of a drug subject to a window of absorption in a conventional controlled release delivery system can lead to subtherapeutic blood levels and ineffective treatment of the diseased state for which the drug was intended.

Drugs with very high solubility in water (for example, greater than 100 mg/ml) can be difficult to formulate into a controlled release oral dosage form. Solubility is a driving force for a drug substance to dissolve in water; the greater the solubility the greater the rate of dissolution when all other factors are maintained constant.

In a controlled release dosage form, the formulator tries to reduce the rate of dissolution by, for example, embedding the drug in a polymeric matrix or surrounding it with a polymeric barrier membrane through which drug must diffuse to be released for absorption. To reduce the rate of release of drug from the dosage form to an appropriate level consistent with the blood level profile desired for a drug possessing very high water solubility, very large amounts of polymer would be required for the matrix or barrier membrane. If the total daily dose of drug to be delivered is of the order of only a few milligrams this may be feasible, but many drugs having the solubility properties described require total daily doses of the order of many hundreds of milligrams. Whilst it is possible to create oral controlled release dosage forms for such products by use of large amounts of polymer, an unacceptably large dosage form may result.

A further problem with highly water soluble drugs formulated into a controlled release dosage form is that a significant and variable "burst" of drug can occur from these systems. The burst of highly water soluble drug is the initial rapid release of drug that occurs from oral controlled release dosage forms when first contacting fluid, such as gastric fluids, prior to release controlling mechanisms of the dosage form establishing themselves and a stable release rate being provided. Hydration of any polymer matrix used to formulate the dosage form is a pre-requirement of establishing a stable release rate. Thus, a readily hydrating polymer is required to establish the desired stable release rate. However, if the polymer used is slow to hydrate, then an undesirable variable burst can occur.

Studies by Vidon et al (1) strongly suggest that there is permeability limited absorption of metformin. Perfusing drug into the jejunum via an intubation technique showed a 2.5 fold greater area under the plasma concentration-time profile (a measure of the quantity of drug absorbed) compared with similar introduction of drug into the ileum. Drug was not detectable in plasma when drug was perfused into the colon. Drug will transit down the small intestine following dissolution from an ingested dosage form and, if absorption rate is slow, it is possible that drug can reach regions of poor permeability before absorption of a given dose is complete. In such a case, increasing the given dose may be predicted to result in a reduction in the percentage of administered dose absorbed.

A dosage form that allows a reduction in dosing frequency, providing patient convenience that would probably improve compliance might achieve improvements in the therapeutic regimes employing metformin. Conventional extended release formulations have been demonstrated to invariably compromise the availability of metformin (2), (2A), and (2B). This is probably because the dosage form carries a significant proportion of the drug content remaining to be released, as the dosage form is carried to regions of the gastrointestinal tract with very poor permeability to the drug. To reduce dosing frequency, the rate of release from the dosage form must be such as to extend effective plasma levels, but the potential for effective delivery at this rate is compromised by the combined influences of the significant reduction in permeability to the drug in passing from the proximal small intestine down to the colon and the limited residence time in the regions of the gastrointestinal tract where the drug is well absorbed. That transit time down the "useful" region of the gastrointestinal tract is only likely to be of the order of a few hours.

Maintained or even improved bioavailability from an extended release dosage form that releases metformin at a rate likely to provide the desired plasma levels of drug for an extended time period might, however, be possible from a dosage form that has extended residence time in the upper gastrointestinal tract, resisting mechanisms that promote normal transit time for solid materials. That this principle might work in practice was demonstrated in an in-house study where metformin was co-administered with propantheline, an agent that reduces gastrointestinal motility. Compared with giving metformin alone, the combination provided an increased AUC, a delayed Tₘₐₓ and an extended time period over which therapeutically beneficial plasma levels of drug were maintained.

Giving a drug such as metformin for the treatment of diabetes with a further drug, such as propantheline, not used for the treatment of diabetes and where the sole intent of using the second agent is to achieve extended residence time in the upper GI tract, has many disadvantages although it is likely to allow effective extended delivery of metformin to an optimal absorption site. The co-administered drug may have other undesirable pharmacological effects or side effects deleterious to the patients well being and detract from the improved quality of life offered by the treatment for their diabetes. Furthermore, it may be difficult or impossible to appropriately co-formulate the two agents due to chemical compatibility issues or solubility differences, the latter preventing the required release rate of agent influencing residence time in the upper GI tract. Thus, the patient could be required to take separate, multiple medications to achieve the desired effect. The timing of taking the two medications would be critical to effective delivery of the drug with the limited window of absorption and many patients may thus fail to take their medication correctly resulting in ineffective treatment of their diabetes.

### Prior Art Gastro-Retentive Systems:

It would be desirable to provide a dosage form that inherently has the property of extended gastric residence, possessing some resistance to the pattern of waves of motility present in the gastrointestinal tract that serve to propel material through it. There have been many attempts to provide for this, with varying degrees of success.

Possible approaches described in prior art include:
1. Floating or buoyant systems:
   These are designed to have a low density and thus should float on gastric contents after administration until the system either disintegrates (and presumably the resultant particles empty from the stomach) or the device absorbs fluid to the point where its density is such that it loses buoyancy and can pass more easily from the stomach with a wave of motility responsible for gastric emptying. Watanabe et al (3) has used low density polystyrene foam particles in which polymer and drug layers were loaded. The disadvantages of such a system may include loss of controlled density so that it does not get out from the stomach, drug loading during manufacture and larger amounts of polymer requirement to retard release incase of water soluble drugs.
   Hydrodynamically balanced systems of capsules and tablets with low density to enable floating on stomach contents have been described by Sheth (4,5,6). These get slowly eroded after administration, losing buoyancy and are flushed down from the stomach.
   For drugs with pH dependent solubility (7), one can combine buoyancy with control of drug release at different pH values to achieve better control and hence dissolution depending on the environment pH.
   However the above technology would be difficult to achieve for larger doses of water soluble drugs and may result in a faster release as larger amounts of polymer are also required.
      A bilayered composition wherein one of the layers generates the buoyancy effect and the other controls the release rate of the active (8) maybe the solution but for high loading drugs this would be a constraint. Ichikawa (9) has described a device consisting of a drug loaded core, surrounded by a gas generating layer, surrounded by a polymeric layer for controlling drug release.
   Clinical success has been limited with these systems due to their dependence on the fluid available in the stomach, their dependence on posture of the patient and their physical size (11). In fact a study by Davis (10) shows no benefits in-vivo between floating and non-floating formulations.
2. Bioadhesive systems:
   These are designed to imbibe fluid following administration such that the outer layer becomes a viscous, tacky material that adheres to the gastric mucosa/mucus layer. This should encourage gastric retention until the adhesive forces are weakened for example by continuing hydration of the outer layer of the device or by the persistent application of shear. Longer et al (12) in J.Pharm Sci., 74, 406-411 (1985) reports use of polycarbophil as a suitable polymer to achieve a prolonged residence time. The polymer here adhere to mucous and not mucosa and as the mucous layer in humans is thinner and sloughs off readily, it might carry away the dosage form too.
3. Swelling and expanding systems:
   These are designed to be sufficiently small on administration so as not to make ingestion of the dosage form difficult. On ingestion they rapidly swell or unfold to a size that precludes passage through the pylorus until after drug release has progressed to a required degree.
   Gradual erosion of the system or its breakdown into smaller particles enables it to leave the stomach.

Other solutions to encouraging prolonged gastric residence have included dosage forms that unfold rapidly within the stomach to a size that resists gastric emptying. Such systems retain their integrity for an extended period and will not empty from the stomach at all until breakdown into small pieces occurs.

An alternate approach to using size to modulate gastric residence of a dosage form is to use a hydrophilic erodible polymer system that is of a convenient size for administration to humans. On imbibing fluid the system swells over a short period of time to a size that will encourage prolonged gastric retention, allowing sustained delivery of contained drug to absorption sites in the upper gastrointestinal tract. Because these systems are made of an erodible and hydrophilic polymer or polymer mixture, they readily erode over a reasonable time period to pass from the stomach. The time period of expansion is such that this will not occur in the esophagus and if the system passes into the intestine in a partially swollen state, the erodibility and elastic nature of the hydrated polymer will eliminate the chance of intestinal obstruction by the device.

Mamajek et al (13), U.S. Pat. No. 4,207,890, describes a system wherein a drug release rate controlling (metering) component and a swelling component are mixed with drug enclosed within a membrane. The swelling component draws in fluid through the membrane, which maintains system integrity during its functioning, and the drug metering component controls the rate of drug release through the membrane.

Urquart (14) describes a different approach, which consists of a matrix of hydrogel that imbibes fluid to swell the system so that it reaches a size encouraging prolonged gastric retention. This matrix surrounds a plurality of tiny pills consisting of drug with a release rate-controlling wall of fatty acid and wax surrounding each of the pills.

Shell (15,16,17) has described systems for delivering drugs for the treatment of diseases of the upper gastrointestinal tract or for delivering drugs that might be irritating or injurious to the gastrointestinal mucosa. A swelling hydrogel polymer has embedded within it drug particles that dissolve once the hydrogel matrix is hydrated. The swollen matrix is of a size to encourage gastric retention but only dissolved drug reaches the mucosa and this can be delivered in a sustained manner. Such a system thus does not insult the mucosa with solid particles of irritant drug and is suitable for delivering drug to upper gastrointestinal tract. These systems only apply in case of drugs of limited water solubility.

In the case of metformin, it is desirable to provide a dosage form that allows extended delivery of the drug and has a prolonged gastric residence via swelling of the system rather than unfolding or expanding of a folded device, and that may be manufactured on a commercial scale. The prolonged gastric residence time is required due to the window of absorption seen with metformin.

Another problem for extended delivery of metformin is its very high water solubility. High levels of polymer would be needed if many prior art approaches to provide the required release rate were employed. This could result in a rapid and variable initial release (burst) of drug from an extended release dosage form. The latter will thus give rise to difficulty in providing a true control of drug release and minimal inter-patient variability in drug plasma levels (arising from the possibility of variable burst of drug from tablets given to different patients).

### Prior Art Controlled Release Systems for Very Soluble Drugs

Typical prior art techniques for creating a controlled release oral dosage form would involve either matrix systems or multi particulate systems. Matrix systems may be formulated by homogeneously mixing drug with hydrophilic polymers, such as hydroxypropyl methyl cellulose, hydroxy propyl cellulose, polyethylene oxide, carbomer, certain methacrylic acid derived polymers, sodium alginate, or mixtures of components selected from these, and compressing the resultant mixture into tablets (employing some other excipients where needed). Hydrophobic polymers, such as ethyl cellulose, certain polymeric methacrylic acid esters, cellulose acetate butyrate, poly (ethylene-co-vinyl-acetate) may be uniformly incorporated with the above materials to give additional control of release. A further alternative involves embedding drug within a wax based tablet, by granulation or simply mixing of drug with a wax, such as carnauba wax, microcrystalline wax or commercially available purified fatty acid esters. As noted above, it may not be possible to use these approaches with very highly water- soluble drugs.

Multi particulate systems consist of a dosage form based on a plurality of drug loaded spheres, prepared by layering drug onto a core, usually a sugar-starch mixture sphere of around 0.8 mm diameter, until a sufficient level is reached, and then providing a drug release barrier around the drug-loaded sphere. Drug-loaded spheres can also be made by wet massing a mixture of drug and excipients, forcing the wet mass through a perforated screen to form short strands which are rounded in a spheronisation apparatus before drying and having the drug release barrier applied. The drug release barrier can be a wax, such as carnauba wax or glyceryl fatty acid esters, or a polymeric barrier, such as a mixture of ethyl cellulose and hydroxypropylmethylcellulose. These work well for moderately soluble drugs with doses in the units of milligrams to less than a few hundred milligrams per day.

In several examples, prior art systems seem to provide a controlled release formulation of a very water- soluble drug by improving the multi particulate system approach. Fisher discloses a multi particulate system for highly soluble drugs especially opiate agonists (18) based on drug containing cores surrounded by a drug release controlling barrier which has the property of being partially soluble at a highly acidic pH.

Hansraj (19) coats drug loaded cores with methacrylic or acrylic acid derived polymers whose properties are modified by inclusion of at least one anionic surfactant. In such a system, drug release of highly water soluble drugs is controlled without having to resort to the use of thick coatings on the release rate controlling layer.

Rollet (20) achieves prolonged release of a drug from a multi particulate formulation based on fine particles of hydrophilic and hydrophobic silicas or silicates. Presumably, this system would function for drugs of high water solubility.

Multi particulate systems are usually filled into capsules to provide unit dose forms because of the damage caused to such particles in trying to compress them into tablets. Total dose contained in a single unit is constrained by the loading possible in a hard gelatin capsule of easily swallowable size and is usually not more than a few hundred milligrams.

Single unit controlled release systems applicable to highly water soluble drugs include the application of multiple layers around a dose form as described by Howard (21). Where coating is not employed, special mixtures of polymers or formation of a complex with the drug have been used. Macrae (22) uses mixtures of polyethylene oxide and hydroxypropylmethylcellulose with optional enteric polymers to produce a constant release rate for highly water soluble drugs. Belenduik (23) combines the highly water soluble drug with a hydrophilic polymer based on acrylic acid and disperses this in a hydrophobic matrix. Variations of Alza osmotic systems have been described suitable for highly water soluble drugs such as venlafaxine hydrochloride (24). These systems need two layers, a drug layer and an osmotically driven displacement layer all surrounded by a water permeable/drug impermeable membrane with an exit passage in this membrane for the drug.

Granules of highly water soluble clavulanate were prepared (25) having to employ a barrier layer of a hydrophobic waxy material in order to provide for controlled release of this material when co-formulated with controlled release amoxycillin trihydrate granules in capsule or compressed tablet.

In accordance with the present invention, a novel way has been found for formulating drug with high water solubility and a limited window of absorption, such as metformin or a salt thereof, in combination with another immediate /sustained release drug such as an anti-diabetic drug belonging to a different class according to the claims in combination with yet another immediate release drug, such as another anti-diabetic drug belonging to yet another different class according to the claims, which has a window of absorption in the upper gastrointestinal tract, to provide a dosage form that inherently has a prolonged gastric residence. This is accomplished without need for a) co-administration of a drug such as propantheline, and (b) low density formulation or gas generation within the formulation. This invented formulation (a) achieves extended gastric residence by virtue of size but it will degrade in vivo so as not to have the potential for causing gastric or intestinal obstruction, and (b) controls drug release adequately where the initial burst of drug is under control. The formulations of the invention will provide for an extended release formulation of drug with minimal inter-patient variability in pharmacokinetic parameters.

In addition, the present invention provides the use for lowering insulin resistance or treating diabetes of the biphasic controlled release formulation of the invention containing an antidiabetic pharmaceutical is administered to a patient in need of treatment.
The antidiabetic pharmaceutical employed is a biguanide, preferably metformin or a pharmaceutically acceptable salt thereof such as the hydrochloride, hydrobromide, fumarate, succinate, p-chlorophenoxy acetate embonate etc, all of which are collectively referred to as metformin. The fumarate and succinate salts are preferably the metformin (2:1) fumarate, and the metformin (2:1) succinate disclosed in U.S. application Ser. No. 09/262,526 filed Mar. 4,1999. Metformin hydrochloride salt is the most preferred drug.

In another aspect of the present invention, a use is provided for lowering insulin resistance or treating diabetes of the biphasic controlled release formulation of the invention which contains metformin and is administered in a dosing regimen of at least one gram metformin, once daily, preferably from about 1 to about 3 grams, once daily, to a patient in need of treatment.

The term "diabetes" as employed herein refers to type 2 diabetes and type 1 diabetes, usually type 2 diabetes.

The term slow-release here applies to any release from a formulation that is slow in nature and includes various terms used interchangeably in the pharmaceutical context like extended release, delayed release, controlled release, timed release, specific release, targeted release etc.....

The term "extended release material" as present in the inner solid particulate phase and the outer solid continuous phase refers to one or more hydrophilic polymers and/or one or more hydrophobic polymers and/or one or more other type hydrophobic materials, such as, for example, one or more waxes, fatty alcohols and/or fatty acid esters. The "extended release material" present in the inner solid particulate phase may be the same as or different from the "extended release material" present in the outer solid continuous phase..

The term "water solubility" or similar term when characterizing a drug, medicament or pharmaceutical in the invention refers to a solubility in water at ambient temperature of at least about 10 mg/ml water, preferably at least about 25 mg/ml water or more, and more preferably greater than 75 mg/ml.

The term "window of absorption" or similar term when characterizing a drug, medicament or pharmaceutical in the invention refers to an oral bioavailability of less than about 50%, usually less than about 45%, usually decreasing with increasing dose, and almost invariably having permeability/transit time limited absorption.

The term candidate for sustained release encompasses all the characteristics of a drug which make it a candidate for formulating it into an extended release fashion like a short elimination half life and consequent dosing of more than once a day, a single dose product given in an extended fashion to achieve better clinical results and avoid side effects associated with an immediate release etc....

The inner solid particulate phase will contain drug in an amount within the range from about 10 to about 98% by weight, preferably from about 15 to about 95% by weight, and extended release material in the form of hydrophilic polymers and/or hydrophobic polymers and/or other hydrophobic material in an amount within the range from about 5 to about 95% by weight, preferably from about 7 to about 85% by weight, the above percentages being based on the weight of the inner solid particulate phase. Where mixtures are employed, the hydrophilic polymer could be employed in a weight ratio to hydrophobic polymer and/or other hydrophobic material within the range from 0.05:1 to about 19:1, preferably from 0.1:1 to about 10:1.

Hydrophilic polymers which may be employed in the inner solid particulate phase and/or outer solid continuous phase include hydroxypropylmethylcellulose, hydroxypropylcellulose, sodium carboxymethylcellulose, carboxymethylcellulose calcium, ammonium alginate, sodium alginate, potassium alginate, calcium alginate, propylene glycol alginate, alginic acid, polyvinyl alcohol, povidone, carbomer, potassium pectate, potassium pectinate, etc

Hydrophobic polymers which may be employed in the inner solid particulate phase and/or outer solid continuous phase include, ethyl cellulose, hydroxyethylcellulose, ammonio methacrylate copolymer (Eudragit RL.TM. or Eudragit RS.TM.), methacrylic acid copolymers (Eudragit L.TM. or Eudragit S.TM.), methacrylic acid-acrylic acid ethyl ester copolymer (Eudragit L 100-5.TM.), methacrylic acid esters neutral copolymer (Eudragit NE 30D.TM.), dimethylaminoethylmethacrylate-methacrylic acid esters copolymer (Eudragit E 100.TM.), vinyl methyl ether/maleic anhydride copolymers, their salts and esters (Gantrez.TM.) etc.

Other hydrophobic materials which may be employed in the inner solid particulate phase and/or outer solid continuous phase include, but are not limited, to waxes such as beeswax, carnauba wax, microcrystalline wax, and ozokerite; fatty alcohols such as cetostearyl alcohol, stearyl alcohol; cetyl alcohol myristyl alcohol etc; and fatty acid esters such as glyceryl monostearate, glycerol monooleate, acetylated monoglycerides, tristearin, tripalmitin, cetyl esters wax, glyceryl palmitostearate, glyceryl behenate, hydrogenated castor oil,etc.

The use of combinations of metformin (a biguanide) and glyburide (a sulfonylurea) has been demonstrated to be synergistic in clinical trials when compared with the use of the individual agents separately (see Physician's Desk Reference 2000, page 832). The monograph also advocates the use of combinations of metformin and sulfonylureas for patients not controlled on metformin alone. Several prior art references pertain to pharmaceutical compositions having combinations of biguanides and sulfonylureas providing for controlled or immediate release of both of the drugs. For example, a unit-dose combination of metformin and glipizide as an immediate release formulation is commercially available (Zidmin™ tablets, Wockhardt), and a combination dosage form of metformin and glyburide for immediate release is described in U.S. Patent No. 6,303,146 to Bonhomme et al. However, a triple combination of drugs such as anti diabetics either as immediate release or as sustained release or a combination as disclosed in the claimed invention are hitherto unknown Furthermore, for the administration of a triple combination of a controlled release drug such as biguanide with a glitazone and a sulfonylurea for synergistic effect in the treatment of NIDDM, the individual commercially available products have been heretofore administered in the present invention together. There is no availability, in clinical practice, of such combinations of a controlled release biguanides along with a controlled / immediate release sulfonylurea and a glitazone for ready administration. The provision made in the current invention, of a controlled release drug such as biguanide alone or in a fixed-dose combination with a controlled / immediate release drug such as, sulfonylurea and an immediate release drug such as glitazone would fill a highly desired gap in the medical armamentarium. Such an invented composition would improve the treatment of diseases such as NIDDM through significantly enhanced patient compliance because of ease of administration and a reduced frequency of dosing. There is also the possibility of a significant reduction in the doses of the drug substances used in combination because of the synergistic action, resulting in a possible reduction in toxicity.

Combinations of biologically active agents are especially difficult to formulate because of the inherent differences in physicochemical properties, the possible drug-drug interactions between the drugs and also in the ingredients used for formulation of the combination composition.

The compositions exemplified for controlled release drugs such as metformin in the prior art references contain very high amounts of polymers (hydrophilic) that upon contact with gastric fluids swell to form a soft gelatinous mass. The release from a delivery system constituting hydrophilic polymers as taught by the prior art is dependent upon the gastric emptying time and extent of the hydration of the polymer. Polymers being hydrophilic in nature hydrate to form a gel layer on exposure to aqueous fluids, which thereafter slowly dissolves to release the medicament. The rate and extent of hydration of the polymer is dependent on the pH of the media. The rate of release of drug from such a system is primarily dependent on viscosity of the polymer, rate of water imbibition, resultant rate of swelling of polymer, drug dissolution and diffusion from the polymer. The release of medicament is also said to take place by leaching action at or near the surface. However, it is well recognized that the application of such a system to obtain a consistent rate of release of the drug wherein it is regulated by the diffusion of the polymer is difficult to maintain.

### SUMMARY OF THE INVENTION

It is therefore an object of the invention to provide efficacious methods for the development of drug delivery systems of triple combination drugs according to the claims. For example biguanides in combination with other drugs according to the claims to treat diabetes-associated maladies. Furthermore, in light of the foregoing, the principal object of the present invention is to provide a delivery system for oral administration of a controlled release drugs used for the treatment and control of various metabolic disorders, diseases associated with humans. Such a triple combination providing glycemic control to diabetic patients include a sustained / controlled / extended release biguanide in combination with a sustained / immediate release drugs such as sulfonylurea and an immediate release drugs such as glitazone with the features of appended claim 1.

It is an object of the present invention to provide an oral delivery system for drugs, wherein atleast one of the drugs is a suitable candidate for controlled / extended release such as a triple combinations of biguanide, sulfonylurea and a glitazone that provides a controlled / sustained release by gastro-retention for at least one of the components of the dosage form according to the claims.

It is an object of the present invention to provide an oral delivery system according to the claims that provides a controlled / sustained release by gastroretention.

It is a further object of the present invention to provide an oral delivery system for the combination of a controlled release drugs with a controlled / immediate release drugs and an immediate release drugs according to the claims.

It is another object of the present invention to provide a delivery system for oral administration constituting of release in the body of a mammal, a sustained release drug biguanide), a sustained / immediate release drug (sulfonylurea) and an immediate release drug (glitazone).

It is also an object of this invention to provide the use of these triple compositions for the treatment of various diseases wherein the components in any order are consumed within 0-12 hours after administration of any of the other two components comprising / formulation.

These objects are achieved by virtue of the present invention, which relates to an oral delivery system that selectively delivers drugs at an optimal rate patients over a period of time during treatment and aims to achieve a reduction in the dose of drugs administered after an initial therapy with this regimen. The reduction in dosage shall be beneficial to the patient and will be at the discretion of the medical doctor depending upon the pathological profile obtained after treatment with this combination.

It is a well-known fact that cardiovascular mortality is two to three times higher in men with diabetes and three to five times higher in women with diabetes than in people without diabetes: Type 2 diabetes and hypertension are commonly associated conditions, both of which carry an increased risk of cardiovascular and renal disease. The prevalence of hypertension in type 2 diabetes is higher than that in the general population, especially in younger patients. At the age of 45 around 40% of patients with type 2 diabetes are hypertensive, the proportion increasing to 60% by the age of 75. Hypertension increases the already high risk of cardiovascular disease associated with type 2 diabetes and is also a risk factor for the development of microalbuminuria and retinopathy.

In the general population treatment to lower blood pressure reduces the incidence of stroke and myocardial infarction, particularly in elderly people. In patients with type 1 diabetes who have microalbuminuria or overt nephropathy strict control of blood pressure reduces urinary albumin excretion and deterioration in renal function. Lowering blood pressure also decreases albuminuria in type 2 diabetes, but whether it also reduces the risk of end stage renal disease or of cardiac disease is not known.

Epidemiologic and experimental data suggest that activation of the renin-angiotensin-aldosterone system has an important role in increasing the risk of cardiovascular events. Angiotensin converting-enzyme inhibitors block the activation of the renin-angiotensin system and could retard the progression of both heart failure and atherosclerosis as discussed by Lonn E et.al (26). Therefore, Angiotensin-converting-enzyme inhibitors may also reduce the risk of stroke, by lowering blood pressure, and may prevent complications related to diabetes.

Other cardiovascular risk factors in people with diabetes include conventional risk factors (age, prior cardiovascular disease, smoking, hypertension, dyslipidemia, sedentary lifestyle, family history of premature cardiovascular disease) and more diabetes specific risk factors (elevated urinary protein excretion, poor glycemic control).

Interventions that can improve cardiovascular outcomes in people with diabetes as discussed by Sigal R et. al (27); include
- Aggressive lowering of blood pressure in people with diabetes who have hypertension reduces cardiovascular morbidity and mortality.
- Aspirin is effective in primary and secondary prevention of cardiovascular events in people with diabetes.
- Statins and fibrates are effective in primary and secondary prevention of cardiovascular disease in people with diabetes and dyslipidemia.

### LESSONS FROM 2 MAJOR STUDIES (HOPE AND UKPDS)(28,29)

The Heart Outcomes Prevention Evaluation study (HOPE) compared ramipril to placebo in people with and without diabetes, who were aged 55 years or older and who had a history of cardiovascular disease or diabetes plus at least one other cardiovascular risk factor. HOPE was stopped 6 months early (after 4.5 years) because of a consistent benefit of ramipril compared with placebo. In the MICRO-HOPE sub-study of 3,577 people with diabetes, ramipril lowered the relative risk of a major cardiovascular event by 25% (myocardial infarction, stroke or cardiovascular death) and the absolute risk by 4.5%. The relative risk of overt nephropathy was reduced by 24% (absolute risk reduction 2.0%). The benefit was apparent irrespective of whether participants had a history of cardiovascular events, hypertension, or microalbuminuria, were taking insulin or oral antidiabetic drugs, or had type 1 or type 2 diabetes (HOPE study Investigators).

The UK Prospective Diabetes Study was the largest and longest study of type 2 diabetes. Its main messages were:
- Type 2 diabetes is a progressive disease and should never be considered a mild form of diabetes.
- Intensive treatment of type 2 diabetes with sulphonylureas and/or insulin resulted in a 25% relative risk reduction in microvascular endpoints.
- Metformin should be the drug of choice for overweight patients, assuming they have no contraindications. Patients assigned metformin had a 32% relative risk reduction of developing any diabetes related end points, both microvascular and macrovascular.
- Tight control of blood pressure reduces the risk of any non fatal or fatal diabetic complication and of death related to diabetes. Deterioration in visual acuity was also reduced making the reduction of blood pressure a high priority in patients with type 2 diabetes.
- Multiple drug combinations may be necessary to achieve blood pressure targets.

### ASPIRIN IN DIABETICS

People with diabetes have a two- to fourfold increase in the risk of dying from the complications of cardiovascular disease. Both men and women are at increased risk. Atherosclerosis and vascular thrombosis are major contributors, and it is generally accepted that platelets are contributory. Platelets from men and women with diabetes are often hypersensitive in vitro to platelet aggregating agents. A major mechanism is increased production of thromboxane, a potent vasoconstrictor and platelet aggregant. Investigators have found evidence in vivo of excess thromboxane release in type 2 diabetic patients with cardiovascular disease. Aspirin blocks thromboxane synthesis by acetylating platelet cyclo-oxygenase and has been used as a primary and secondary strategy to prevent cardiovascular events in nondiabetic and diabetic individuals. Meta-analyses of these studies and large-scale collaborative trials in men and women with diabetes support the view that low-dose aspirin therapy should be prescribed as a secondary prevention strategy, if no contraindications exist. Substantial evidence suggests that low-dose aspirin therapy should also be used as a primary prevention strategy in men and women with diabetes who are at high risk for cardiovascular events; Colwell JA et.al. (30).

Treatment with aspirin was associated with a significant reduction in cardiac and total mortality among non-insulin-dependent diabetic patients with coronary artery disease. The absolute benefit of aspirin was greater in diabetic patients than in those without diabetes as discussed in David Harpaz et. al. (31).

A meta-analysis of randomized trials of anti-platelet therapy for prevention of death, myocardial infarction, and stroke in various categories of patients suggests that there was a significant reduction in vascular events (one quarter in each of these four main categories) and were separately statistically significant in middle age and old age, in men and women, in hypertensive and normotensive patients, and in diabetic and non diabetic patients

### ADVANTAGES OF TRIPLE COMBINATION

1. Reduced incidence of cardiovascular and renal complications of diabetes
2. Reduced morbidity and mortality
3. Once a day administration
4. Improved compliance

### METFORMIN XL (Extended Release)

Metformin hydrochloride promotes glucose lowering by reducing hepatic glucose production and gluconeogenesis and by enhancing peripheral glucose uptake. Metformin XL is a modified release gastro-retentive formulation. By virtue of its gastro-retentive property it releases Metformin gradually in small amounts, which is well absorbed in the upper part of the small intestine and duodenum. Metformin incorporated into the gastro-retentive formulation is released slowly over a prolonged period of 24 hours; hence given once a day.

Metformin XL has distinct advantages over plain Metformin, which are as follows:
1. It reduces the number of daily doses and increases patient compliance. As treatment of diabetes is life-long, this aspect is very important from a patient's point of view.
2. Metformin XL, being a modified release preparation can also avoid "dose-loading". This commonly occurs with conventional oral formulations when large doses are given which may cause sudden release and absorption of a large amount of drug.
3. Metformin XL is released in smaller doses in upper part of the small intestine, and hence ensures increased bioavailability and decreased side effects. In contrast, conventional Metformin has lesser bioavailability since its absorption decreases as it passes through the lower part of small intestine.
4. Conventional Metformin has an oral bioavailability of 40 to 60 % and gastrointestinal absorption is apparently complete within 6 hours of ingestion. Plasma t 1/2 is 2 to 6 hours. Hence it has to be given 2 to 3 times a day, whereas Metformin XL being a controlled release "gastro-retentive" formulation, is released in small quantities in upper part of small intestine where the drug is better absorbed and has a prolonged duration of action (24 hours).
5. Metformin XL - the absorption is more dependable and complete as the drug is released gradually mainly in the upper part of small intestine, whereas in Metformin plain the absorption is erratic as Metformin is also absorbed in the latter part of small intestine where absorption is erratic and "non-dependable".
6. Since Metformin XL is released slowly, side-effects like flatulence, abdominal discomfort, diarrhea and lactic acidosis are less unlike plain Metformin.
7. An inverse relationship was observed between the dose ingested and relative absorption with therapeutic doses ranging from 0.5 to 1.5 gms suggesting the involvement of an active, saturable absorption process. Thus an extended release formulation of Metformin can not only optimize the daily requirement of Metformin, but can also reduce the need of a higher dose.

### DESCRIPTION OF THE INVENTION

In accordance with the present invention, a novel therapeutic triple combination drug delivery system by gastro-retention of different classes of drugs according to the claims is addressed with anti-diabetic. Anti-diabetic drugs according to the claims given in a sustained and / or immediate release belonging to different classes of anti-diabetic agents, which act by different mechanisms of action, resulting in a better level of glycemic control is described.

A better control and patient compliance is achieved with a pharmaceutical composition according to claim 1 by:
a) Using a sustained / controlled release agent which is a candidate for a sustained release regimen with
b) An immediate / controlled release agent which is a candidate for an immediate release or controlled release as the case maybe with
c) An immediate release drug preferably having a long elimination half life.

The controlled release is achieved through use of a hydrophilic polymer and /or one or more hydrophobic polymers and /or one or more hydrophobic materials.

The immediate release component may be coated onto the extended release components(s) or may be compressed onto it

### The First Component:

The sustained / controlled release component employed belongs to the class of a soluble, absorption window drugs which fulfil the characteristics find clinical rationale for a sustained / controlled release and its pharmaceutically acceptable salts, presently a biguanide.

### The Second Component:

The immediate release or sustained / controlled release second component employed belongs to the class of drugs wherein immediate release uses drugs with a long elimination half-life and sustained / controlled release uses drugs with a short elimination half life and its salts, presently a sulfonylurea.

### The Third Component:

An immediate release component belonging to the class not covered by the classes of the first and second components and it is preferably a drug with a long elimination half life, presently a glitazone.

The ranges of the different active components and the amounts they make up as a part of the triple combination as a percentage of the total weight of components given as a single / dual or triple combination therapy or as a kit are given below as an example:
First active component: Slow release:10 - 90% w/w of the total formulation.
Second active component: Immediate release- 0.025 - 0.5% w/tw of total formulation.
   Controlled release- 0.25 -10% w/w of total formulation.
Third active component: Immediate release 0.25 - 5% w/w of total fomulation.

The hydrophilic polymers which may be employed for achieving the sustained / controlled release include but not limited to hydroxypropylmethylcellulose, hydroxypropylcellulose, sodium carboxymethylcellulose, carboxymethylcellulose inorganic salts, alginate and their salts, pectates and pectinates, povidone, gelatin and its commercially available physicochemical forms like Gelatin 180 bloom, alginates etc.

Hydrophobic polymers employed to achieve sustained / controlled release includes but not limited to ethyl cellulose, hydroxyethylcellulose etc and other acrylic acid copolymers.

Cyclodextrins may also be used to form an inclusion complex for one of the active agents in the sustained / controlled release component(s) as an inclusion complexer to achieve this. It may additionally in complex form help to achieve compatability between the actives in case of any incompatability between the two actives by physically separating them within the formulation through formation of a complex. One may use an unsubstituted, or substituted cyclodextrin.

Typical examples for the formulations for this triple drug combination and a brief process of manufacture to illustrate the present invention is given below for illustrative purposes:

**Metformin HCl XL + Glipizide XL + Pioglitazone IR tablets.**

| **Ingredients** | **% w/w with respect to total weight** | **Function** |
|---|---|---|
| Metformin Hydrochloride | 51.6 | Active (Biguanide) |
| HPMC K100M | 1.3 | Hydrogel |
| HPMC K15M | 3.7 | Hydrogel |
| Povidone K 90 | 2.4 | Binder |
| Magnesium stearate | 0.6 | Lubricant |
| Ethyl cellulose | 1.4 | Rate controlling membrane |
| Polyethylene Glycol 4000 | 0.5 | Plasticizer |
| HPMC E5 | 2.3 | Film former |
| Titanium dioxide | 0.5 | Opacifier |
| Glipizide | 0.5 | Active (Sulfonylurea) |
| Betacyclodextrin | 9.4 | Inclusion complexer |
| HPC LF (Klucel LF) | 0.6 | Hydrogel |
| Sodium lauryl sulphate | 0.8 | Surfactant |
| Dicalcium phosphate | 7.5 | Filler / excipient |
| Stearic acid | 0.2 | Lubricant |
| Colloidal silicon dioxide | 0.2 | Bridging agent |
| Simethicone emulsion | 0.05 | Anti-foaming agent |
| Hydroxyethylcellulose (Natrosol 250M) | 3 | Coating agent |
| Pioglitazone Hydrochloride | 2.2 | Active (Glitazone) |

The above manufacture involves the following steps:
Granulation of Glipizide: Dry mix Glipizide, HPC LF (Klucel LF), beta cyclodextrin and dicalcium phosphate. Granulate with a mixture of surfactant and an antifoaming agenyt in a suitable mixer. After a slight wet mass has been formed add HEC (Natrosol 250M) and mix further for about 5 minutes. Pass the wet granules through a multimill with an aperture of about 6 mm and dry preferably in a fluid bed dryer at a temperature not exceeding 95 deg C. Pass the dried granules through a suitable mesh (preferably 20 mesh) and mill the retained granules through a 2.5mm multi mill preferably. Lubricate the granules obtained with Stearic acid, Magnesium stearate and colloidal silicone dioxide in a suitable blender.
   I. Granulation of Metformin: Dry mix together Metformin HCl, HPMC K 15M, Povidone K 90 in a suitable mixer. Granulate with purified water and dry the granules so obtained in a Fluid Bed Dryer at a temperature not exceeding 95 deg C. Pass the dried granules through a suitable mesh (preferably 20 mesh) and mill the retained granules through a 2.5mm multi mill preferably. Lubricate the granules obtained with Magnesium stearate in a suitable blender.
   II. Mix granulate a and b from above with HPMC K 100M using a suitable blender.
   III. Compress using tablet tooling into a moldable shape.
   IV. Seal coating: Seal coat the compressed tablets using ethylcellulose, HPMC , PEG 4000 and Titanium dioxide
   V. Drug Coating: Coat the immediate release component over the seal coat using a suitable excipient like Opadry in conjunction with the active agent in a suitable solution to achieve the desired loading of the immediate release active agent in the formulation.

**Metformin HCl XL + Gliclazide XL + Pioglitazone IR tablets.**

| **Ingredients** | **% w/w with respect to total weight** | **Function** |
|---|---|---|
| Metformin Hydrochloride | 51.6 | Active (Biguanide) |
| Gliclazide | 1.3 | Active (Sulfonylurea) |
| HPMC K100M | 3.7 | Hydrogel |
| Povidone K 90 | 2.4 | Binder |
| Magnesium stearate | 0.6 | Lubricant |
| Gelatin (180 bloom) | 1.4 | Retardant |
| HPMC E6 | 0.5 | Rate controlling membrane |
| Ethyl cellulose | 2.3 | Film former |
| Polyethylene glycol 4000 | 0.5 | Lubricant |
| Titanium dioxide | 0.5 | Opacifier |
| Pioglitazone HCl | 9.4 | Active (Glitazone) |
| Opadry | 0.6 | Coating agent |

The above manufacture involves the following steps:
a. Granulation of Gliclazide and Metformin: Dry mix Gliclazide, Metformin HCl, HPMC K 100M and Povidone K 90 in a suitable mixer. Granulate with a gelatin dissolved in hot water in a suitable mixer. Dry preferably in a fluid bed dryer at a temperature not exceeding 95deg C. Pass the dried granules through a suitable mesh (preferably 20 mesh) and mill the retained granules through a 2.5mm multi mill preferably. Lubricate the granules obtained with Magnesium stearate in a suitable blender.
b. Compress using tablet tooling into a moldable shape.
c. Seal coating: Seal coat the compressed tablets using ethylcellulose, HPMC , PEG 4000 and Titanium dioxide.
d. Drug Coating: Coat the immediate release component over the seal coat using a suitable excipient like Opadry in conjunction with the active agent in a suitable solution to achieve the desired loading of the immediate release active agent in the formulation.

**Metformin HCl XL + Glimepiride IR + Pioglitazone IR tablets.**

| **Ingredients** | **% w/w with respect to total weight** | **Function** |
|---|---|---|
| Metformin Hydrochloride | 47.2 | Active (Biguanide) |
| Carboxymethylcellulose Sodium (High Viscocity) | 10.4 | Matrix for controlled / sustained release |
| HPMC K100M | 18.9 | Hydrogel |
| Gelatin (180 bloom) | 3.8 | Matrix for controlled / sustained release |
| Microcrystalline cellulose | 9.4 | Filler |
| Magnesium stearate | 0.95 | Lubricant |
| Pioglitazone HCl | 1.98 | Active (Glitazone) |
| Glimepiride | 0.29 | Active (Sulfonylurea) |
| HPMC E6 | 2.83 | Film former |
| Polyethylene glycol 4000 | 0.28 | Plasticizer |
| Titanium dioxide | 0.28 | Opacifier |
| Povidone K 30 | 3.77 | Binder |

The above manufacture involves the following steps:
a. Granulation of Metformin: Dry mix Metformin HCl, HPMC K 100M, sodium carboxymethylcellulose, microcrystalline cellulose and Povidone K 90 in a suitable mixer. Granulate with a gelatin dissolved in hot water in a suitable mixer. Dry preferably in a fluid bed dryer at a temperature not exceeding 95deg C. Pass the dried granules through a suitable mesh (preferably 20 mesh) and mill the retained granules through a 2.5mm multi mill preferably. Lubricate the granules obtained with Magnesium stearate in a suitable blender.
b. Compress using tablet tooling into a moldable shape.
c. Seal coating: Seal coat the compressed tablets using, HPMC , PEG 4000 and Titanium dioxide.
d. Drug Coating: Coat the immediate release component(s) over the seal coat using a suitable excipient like hydroxypropylmethylcellulose and PEG 4000 in a suitable solution to achieve the desired loading of the immediate release active agent(s) in the formulation.

**Metformin HCl XL + Glimepiride IR + Pioglitazone IR tablets.**

| **Ingredients** | **% w/w with respect to total weight** | **Function** |
|---|---|---|
| Metformin Hydrochloride | 77.0 | Active (Biguanide) |
| HPMC K100M | 5.44 | Matrix for controlled / sustained release |
| Povidone K 90 | 3.62 | Hydrogel |
| Magnesium stearate | 0.45 | Matrix for controlled / sustained release |
| Gelatin (180 bloom) | 4.53 | Filler |
| HPMC E6 | 1.81 | Lubricant |
| Ethylcellulose | 1.09 | Active (Glitazone) |
| Polyethylene glycol 4000 | 0.34 | Active (Sulfonylurea) |
| Titanium dioxide | 0.34 | Film former |
| Pioglitazone HCl | 1.9 | Plasticizer |
| Glimepiride | 0.27 | Opacifier |
| Opadry 03B57658 Grey | 3.17 | Binder |

The above manufacture involves the following steps:
a. Granulation of Metformin: Dry mix Metformin HCl, HPC K 100M, and Povidone K 90 in a suitable mixer. Granulate with a gelatin dissolved in hot water in a suitable mixer. Dry preferably in a fluid bed dryer at a temperature not exceeding 95deg C. Pass the dried granules through a suitable mesh (preferably 20 mesh) and mill the retained granules through a 2.5mm multi mill preferably. Lubricate the granules obtained with Magnesium stearate in a suitable blender.
b. Compress using tablet tooling into a moldable shape.
c. Seal coating: Seal coat the compressed tablets using ethylcellulose, HPMC, PEG 4000 and Titanium dioxide.
d. Drug Coating: Coat the immediate release component(s) over the seal coat using a suitable excipient like Opadry Grey in a suitable solution to achieve the desired loading of the immediate release active agent(s) in the formulation.

When tested for in-vitro release, it was observed that around 30-50 % of the drug was released for the sustained / controlled release components within a period of about 2 to 3 hours and not less than 75% was released within a period of about 10 - 12 hours.

It was also seen that the sustained / controlled release component of the invention the time required to achieve T max increased and the Cmax decreased as compared to the immediate release formulations of the respective components given alone but the area under the plasma time concentration curve were not significantly different compared to the immediate release formulation for the respective components.

The claimed new triple combination of various classes of triple combinations such as anti-diabetic agents thus represent a significant advance in the once-a-day administration of therapy for people suffering from diseases such as diabetes, cardiovasular and their related maladies.

Thus one can see that once daily dosing with the present invention, increases patient compliance and represent a significant advance in a triple combination to humans in the treatment of different diseases.

### REFERENCES

1. Vidon, N., Chaussade, S., Noel, M., Franchisseur, C., Huchet, B., Bernier, J. J. (1988), Diabetes Research and Clinical Practice, 4, 223-239.
2. Pentikainen, P. J. (1986), International Journal of Clinical Pharmacology, Therapy and Toxicology, 24, 213-220.
   2A. Noel, D. S. (1980), Kinetic study of normal and sustained release dosage forms of metformin in normal subjects, Journal of International Biomedical Information and Data, 1980, pp. 9 to 20.
   2B. Karttunen et al (1983), The pharmacokinetics of metformin : a comparison of the properties of a rapid-release and a sustained-release preparation, Int. J. Clin. Pharmacology, Therapy and Toxicology, Vol. 21, No. 1, pp. 31-36.
3. Watanabe, S., Kayano, M., Ishino, Y., Miyao, K. (1976), U.S. Pat. No. 3,976,764.
4. Sheth, P., Tossounian, J. L. (1979), U.S. Pat. No. 4,140,755.
5. Sheth, P., Tossounian, J. L. (1978), U.S. Patent 4,126,672.
6. Sheth, P., Tossounian, J. L. (1979), U.S. Pat. No. 4,167,558.
7. Dennis, A. B., Lee, K., Timmins, P. (1992), U.S. Pat. No. 5,169,638.
8. Franz, M. R., Oth, M. P. (1993), U.S. Pat. No. 5,232,704.
9. Ishikawa, M., Miyaka, Y., Watanabe, S. (1989), U.S. Pat. No. 4,844,905.
10. Davis, S. S., Stockwell, A. F., Taylor, J. J., Hardy, J. G., Whalley, D. R., Wilson, C. G., Bechgaard, H., Christensen, F. N. (1986) Pharm. Res., 3, 208-213.
11. Timmermans, J., Moes, A. (1994), J. Pharm. Sci., 83, 18-24.
12. Longer et. al; (1985) J. Pharm. Sci., 74, 406-411.
13. Mamajeck et. al; (1980) U.S.Pat.No. 4,207,890.
14. Urquart, J., Theeuwes, F. (1984), U.S. Pat. No. 4,434,153.
15. Shell, J. W. (1990), U.S. Pat. No. 5,007,790.
16. Shell, J. W. (1993), World Patent WO 9318755.
17. Shell, J. W. (1996), U.S. Pat. No. 5,582,837.
18. Fisher, M. C., Morella, A. M. (1994), European Patent 609961.
19. Hansraj, B. R., Bashir, R. H. (1992), European Patent 502642.
20. Rollet, M. (1987), French Patent 2594693.
21. Howard, S. A., Kotwal, P. M. (1997) U.S. Pat. No. 5,645,858.
22. Macrae, R. J., Smith J. S. (1997), World Patent WO 9718814.
23. Belenduik, G. W., McCarty, J. A., Rudnic, E. M. (1996), U.S. Pat. No. 5,484,608.
24. Bhatti, G. K., Edgren, D. E., Hatamkhani, Z., Wong, P. S., Wong, P. S. L. (1994), World Patent WO 9427589.
25. Palepu, N. R., Venkatesh, G. M., (1997) European Patent 701436.
26. Lonn EM, et al. Circulation 1994;90:2056-2069
27. Sigal R et al: Cardiovascular diseases in Diabetes. Clinical evidence issue 4, BMJ Publishing group, December 2000.
28. HOPE study Investigators. Lancet 200: 355: 253-259
29. HOPE study Investigators. NEJM 2000; 342:145-53
30. Colwell JA. Diabetes Care 20:1767-1771, 1997
31. David Harpaz et al. AM J Med 1998: 105: 494-499

## Claims

1. A pharmaceutical composition comprising
A) a slow release therapeutic agent comprising a biguanide and a hydrophilic and/or at least one or more hydrophobic polymers and/or at least one or more hydrophobic materials,
B) a slow or immediate release therapeutic agent comprysing a sulfonyl urea and, for slow release, a hydrophilic and/or at least one or more hydrophobic polymers and/or at least one or more hydrophobic materials, and
C) an immediate release component comprising glitazone
wherein the pharmaceutical composition is a fixed dose combination for use in the treatment of diabetes and its associated disorders.

2. Pharmaceutical composition for use according to claim 1, wherein the biguanide is selected from the group consisting of metformin, its hydrochloride, hydrobromide, fumarate, succinate, and p-chlorophenoxy acetate embonate.

3. Pharmaceutical composition for use according to claim 1 or 2, wherein the sulfonyl urea is selected from the group consisting of glipizide, glimiperide, glyburide, glibomuride, glisoxepide, gliclazide acetohexamide, chlorpropamide, tolazamide, and tolbutamide.

4. Pharmaceutical composition for use according to any one of claims 1 to 3, wherein the glitazone is selected from the group consisting of rosiglitazone, troglitazone and pioglitazone.

5. Pharmaceutical composition for use according to any one of claims 1 to 4, wherein the hydrophylic polymer is selected from the group consisting of hydroxypropylmethylcellulose, hydroxypropylcellulose, sodium carboxymethylcellulose, carboxymethylcellulose calcium, ammonium alginate, sodium alginate, potassium alginate, calcium alginate, propylene glycol alginate, alginic acid, polyvinyl alcohol, povidone, carbomer, potassium pectate, and potassium pectinate.

6. Pharmaceutical composition for use according to any one of claims 1 to 5, wherein the at least one hydrophobic polymer is selected from the group consisting of ethyl cellulose, hydroxyethylcellulose, ammonio methacrylate copolymer, methacrylic acid copolymers, methacrylic acid-acrylic acid ethyl ester copolymer, methacrylic acid esters neutral copolymer, dimethylaminoethylmethacrylate-methacrylic acid esters copolymer, vinyl methyl ethedmaleic anhydride copolymers, their salts and esters.

7. Pharmaceutical composition for use according to any of claims 1 to 6, wherein the one or more other hydrophobic material(s) is (are) selected from the group consisting of waxes such as beeswax, carnauba wax, microcrystalline wax, and ozokerite; fatty alcohols such as cetostearyl alcohol, stearyl alcohol; cetyl alcohol myristyl alcohol etc; and fatty acid esters such as glyceryl monostearate, glycerol monooleate , acetylated monoglycerides, tristearin, tripalmitin, cetyl esters wax, glyceryl palmitostearate, glyceryl behenate, andhydrogenated castor oil.

8. Pharmaceutical composition for use according to any one of claims 1 to 7 wherein the slow release formulation or the slow release formulations include a mixture of at least a hydrophilic and at least a hydrophobic polymer, particularly in the range from 0.05:1 to 19:1, more particularly in the range from 0.1:1 to 10:1.

9. Pharmaceutical composition for use according to any one of claims 1 to 8, wherein the active component of A) is the first active component and is contained in an amount of 10 to 90 % w/w of the total formulation, the active component of B) is the second active component and contained in an amount of 0.025 to 0.5 % of the total formulation in case of an immediate release formulation and in an amount of 0.25 to 10 % w/w in case of a controlled release formulation of the second component, and the active component in C) is the third active component and is contained in an amount of 0.25 to 5 % w/w of the total formulation.

## Patentansprüche

1. Pharmazeutische Zusammensetzung, umfassend:
A) ein Therapeutikum mit langsamer Freisetzung, umfassend ein Biguanid und ein hydrophiles und/oder wenigstens ein oder mehrere hydrophobe Polymere und/oder wenigstens ein oder mehrere hydrophobe Materialien,
B) ein Therapeutikum mit langsamer oder sofortiger Freisetzung, umfassend Sulfonylharnstoff und, für die langsame Freisetzung, ein hydrophiles und/oder wenigstens ein oder mehrere hydrophobe Polymere und/oder wenigstens ein oder mehrere hydrophobe Materialien, und
C) einen Bestandteil mit sofortiger Freisetzung, umfassend Glitazon,
wobei die pharmazeutische Zusammensetzung eine fixe Dosiskombination zur Verwendung für die Behandlung von Diabetes und damit verbundenen Erkrankungen ist.

2. Pharmazeutische Zusammensetzung zur Verwendung nach Anspruch 1, wobei das Biguanid ausgewählt ist aus der Gruppe, bestehend aus Metformin, dessen Hydrochlorid, Hydrobromid, Fumarat, Succinat und p-Chlorphenoxyacetatembonat.

3. Pharmazeutische Zusammensetzung zur Verwendung nach Anspruch 1 oder 2, wobei der Sulfonylharnstoff ausgewählt ist aus der Gruppe, bestehend aus Glipizid, Glimiperid, Glyburid, Glibomurid, Glisoxepid, Gliclazidacetohexamid, Chlorpropamid, Tolazamid, und Tolbutamid.

4. Pharmazeutische Zusammensetzung zur Verwendung nach einem der Ansprüche 1 bis 3, wobei das Glitazon ausgewählt ist aus der Gruppe, bestehend aus Rosiglitazon, Troglitazon und Pioglitazon.

5. Pharmazeutische Zusammensetzung zur Verwendung nach einem der Ansprüche 1 bis 4, wobei das hydrophile Polymer ausgewählt ist aus der Gruppe, bestehend aus Hydroxypropylmethylcellulose, Hydroxypropylcellulose, Natriumcarboxymethylcellulose, Carboxymethylcellulose-Calcium, Ammoniumalginat, Natriumalginat, Kaliumalginat, Calciumalginat, Propylenglycolalginat, Alginsäure, Polyvinylalkohol, Povidon, Carbomer, Kaliumpectat und Kaliumpectinat.

6. Pharmazeutische Zusammensetzung zur Verwendung nach einem der Ansprüche 1 bis 5, wobei das wenigstens eine hydrophobe Polymer ausgewählt ist aus der Gruppe, bestehend aus Ethylcellulose, Hydroxyethylcellulose, Ammoniummethacrylat-Copolymer, Methacrylsäure-Copolymeren, Methacrylsäure-Acrylsäureethylester-Copolymer, Methacrylsäureester-neutrales Copolymer, Dimethylaminoethylmethacrylat-Methacrylsäureester-Copolymer, Vinylmethylethed-Maleinsäureanhydrid-Copolymeren, deren Salzen und Estern.

7. Pharmazeutische Zusammensetzung zur Verwendung nach einem der Ansprüche 1 bis 6, wobei das eine oder die mehreren weitere(n) hydrophobe(n) Material(ien) ausgewählt ist (sind) aus der Gruppe, bestehend aus Wachsen, wie Bienenwachs, Carnaubawachs, mikrokristallinem Wachs und Ozokerit; Fettalkoholen, wie Cetostearylalkohol, Stearylalkohol; Cetylalkohol, Myristylalkohol, etc; und Fettsäureestern, wie Glycerylmonostearat, Glycerolmonooleat, acetylierten Monoglyceriden, Tristearin, Tripalmitin, Cetylesterwachs, Glycerylpalmitostearat, Glycerylbehenat und hydriertem Rizinusöl.

8. Pharmazeutische Zusammensetzung zur Verwendung nach einem der Ansprüche 1 bis 7, wobei die Formulierung mit langsamer Freisetzung oder die Formulierungen mit langsamer Freisetzung eine Mischung aus wenigstens einem hydrophilen und wenigstens einem hydrophoben Polymer einschließen, bevorzugt in einem Bereich von 0,05:1 bis 19:1, stärker bevorzugt in dem einem Bereich von 0,1:1 bis 10:1.

9. Pharmazeutische Zusammensetzung zur Verwendung nach einem der Ansprüche 1 bis 8, wobei der aktive Bestandteil von A) der erste aktive Bestandteil ist und in einer Menge von 10 bis 90 % w/w der gesamten Formulierung enthalten ist, der aktive Bestandteil von B) der zweite aktive Bestandteil ist und in einer Menge von 0,025 bis 0,5 % der gesamten Formulierung im Fall einer Formulierung mit sofortiger Freisetzung enthalten ist und in einer Menge von 0,25 bis 10 % w/w im Fall einer Formulierung mit gesteuerter Freisetzung des zweiten Bestandteils, und der aktive Bestandteil von C) der dritte aktive Bestandteil ist und in einer Menge von 0,25 bis 5 % w/w der gesamten Formulierung enthalten ist.

## Revendications

1. Composition pharmaceutique comprenant
A) un agent thérapeutique à libération lente comprenant un biguanide et un polymère hydrophile et/ou au moins un ou plusieurs polymères hydrophobes et/ou au moins une ou plusieurs substances hydrophobes,
B) un agent thérapeutique à libération lente ou immédiate comprenant une sulfonyl urée et, pour la libération lente, un polymère hydrophile et/ou au moins un ou plusieurs polymères hydrophobes et/ou au moins une ou plusieurs substances hydrophobes, et
C) un composant à libération immédiate comprenant de la glitazone,
la composition pharmaceutique étant une combinaison de doses fixes destinée à être utilisée dans le traitement du diabète et des troubles y étant associés.

2. Composition pharmaceutique destinée à être utilisée selon la revendication 1, dans laquelle le biguanide est choisi dans le groupe constitué par la metformine, son chlorhydrate, son bromhydrate, son fumarate, son succinate et son p-chlorophénoxy acétate embonate.

3. Composition pharmaceutique destinée à être utilisée selon la revendication 1 ou 2, dans laquelle la sulfonyl urée est choisie dans le groupe constitué par le glipizide, le glimipéride, le glyburide, le glibomuride, le glisoxepide, le gliclazide, l'acétohexamide, le chlorpropamide, le tolazamide et le tolbutamide.

4. Composition pharmaceutique destinée à être utilisée selon l'une quelconque des revendications 1 à 3, dans laquelle la glitazone est choisie dans le groupe constitué par la rosiglitazone, la troglitazone et la pioglitazone.

5. Composition pharmaceutique destinée à être utilisée selon l'une quelconque des revendications 1 à 4, dans laquelle le polymère hydrophile est choisi dans le groupe constitué par l'hydroxypropylméthylcellulose, l'hydroxypropylcellulose, la carboxyméthylcellulose sodique, la carboxyméthylcellulose calcique, l'alginate d'ammonium, l'alginate de sodium, l'alginate de potassium, l'alginate de calcium, l'alginate de propylène glycol, l'acide alginique, l'alcool polyvinylique, la povidone, le carbomère, le pectate de potassium et le pectinate de potassium.

6. Composition pharmaceutique destinée à être utilisée selon l'une quelconque des revendications 1 à 5, dans laquelle le ou les polymères hydrophobes sont choisis dans le groupe constitué par l'éthyl cellulose, l'hydroxyéthylcellulose, un copolymère ammonio-méthacrylate, des copolymères d'acide méthacrylique, un copolymère d'acide méthacrylique-ester éthylique de l'acide acrylique, un copolymère neutre d'esters de l'acide méthacrylique, un copolymère d'esters de l'acide diméthylaminoéthylméthacrylate-méthacrylique, des copolymères vinyl méthyl éther/anhydride maléique, leurs sels et leurs esters.

7. Composition pharmaceutique destinée à être utilisée selon l'une quelconque des revendications 1 à 6, dans laquelle la ou les autres substances hydrophobes sont choisies dans le groupe constitué par les cires telles que la cire d'abeille, la cire de carnauba, la cire microcristalline et l'ozocérite ; les alcools gras tels que l'alcool cétostéarylique, l'alcool stéarylique ; l'alcool cétylique, l'alcool myristylique, etc. ; et les esters d'acides gras tels que le monostéarate de glycéryle, le monooléate de glycérol, les monoglycérides acétylés, la tristéarine, la tripalmitine, la cire d'esters cétyliques, le palmitostéarate de glycéryle, le béhénate de glycéryle, l'huile de ricin hydrogénée, etc.

8. Composition pharmaceutique destinée à être utilisée selon l'une quelconque des revendications 1 à 7, dans laquelle la formulation à libération lente ou les formulations à libération lente comprennent un mélange d'au moins un polymère hydrophile et d'au moins un polymère hydrophobe, particulièrement dans la plage de 0,05/1 à 19/1, plus particulièrement dans la plage de 0,1/1 à 10/1.

9. Composition pharmaceutique destinée à être utilisée selon l'une quelconque des revendications 1 à 8, dans laquelle le composant actif de A) est le premier composant actif et est contenu en une quantité de 10 à 90 % pds/pds de la formulation totale, le composant actif de B) est le deuxième composant actif et est contenu en une quantité de 0,025 à 0,5 % de la formulation totale dans le cas d'une formulation à libération immédiate et en une quantité de 0,25 à 10 % pds/pds dans le cas d'une formulation à libération contrôlée du deuxième composant, et le composant actif dans C) est le troisième composant actif et est contenu en une quantité de 0,25 à 5 % pds/pds de la formulation totale.
